Europäisches Patentamt

**European Patent Office**

**Office européen des brevets**

(19)

(11) Veröffentlichungsnummer: **0 193 163**

**A2**

(12) ## EUROPÄISCHE PATENTANMELDUNG

(21) Anmeldenummer: **86102418.0**

(22) Anmeldetag: **25.02.86**

(51) Int. Cl.⁴: **G 01 N 21/47**
**G 01 N 21/55**

(30) Priorität: **26.02.85 DE 3506690**

(43) Veröffentlichungstag der Anmeldung:
**03.09.86 Patentblatt 86/36**

(84) Benannte Vertragsstaaten:
**AT BE CH FR GB IT LI NL**

(71) Anmelder: **Scheller, Klaus**
**Königsteiner Strasse 47**
**D-6232 Bad Soden(DE)**

(72) Erfinder: **Scheller, Klaus**
**Königsteiner Strasse 47**
**D-6232 Bad Soden(DE)**

(74) Vertreter: **Meier, Robert, Dipl.-Ing.**
**Patentanwalt Dipl.-Ing. Robert Meier Auf dem Mühlberg**
**16**
**D-6000 Frankfurt/Main(DE)**

(54) Vorrichtung zur Ermittlung einer jeweils akuten Sonnenschutzfaktorzahl.

(57) Bei einer Vorrichtung zur Ermittlung einer jeweils akuten Sonnenschutzfaktorzahl zwecks Auswahl des zum Schutze der Haut geeigneten Sonnenschutzmittels ist in einem Gehäuse mit einer Anzeigeeinrichtung für die Sonnenschutz-faktorzahl eine Energiequelle. vorzugsweise eine Batterie, ein Einschalter sowie eine auf den Braunheitswert der Haut ansprechende Sensoreinrichtung mit einem Meßwertgeber vorgesehen, der über eine elektronische Auswertschaltung mit der Anzeigeeinrichtung in Schaltverbindung steht. Die Anzeigevorrichtung kann außerdem Angaben über die Zeit in Minuten machen, die eine Person ohne Haut- bzw. Sonnenschutzcremes sich der Sonne aussetzen kann.

Fig.10

EP 0 193 163 A2

## Vorrichtung zur Ermittlung einer jeweils akuten Sonnenschutz-
## faktorzahl

Die Erfindung bezieht sich auf eine Vorrichtung zur Ermittlung einer jeweils akuten Sonnenschutzfaktorzahl zwecks Auswahl des zum Schutz der Haut geeigneten Sonnenschutzmittels.

Beim Sonnenbaden, aber auch unter Bestrahlungseinrichtungen, z.B. Höhensonnen o.dgl., stehen viele Menschen vor dem Problem, einerseits ihre Haut vor den schädlichen Wirkungen der UV-Strahlen zu schützen, andererseits aber gleichwohl in relativ kurzer Zeit eine gesunde Bräunung der Haut zu erreichen.

Viele verwendete Hilfsmittel sind Sonnenschutzmittel, die gegenüber der UV-Strahlung eine Filterwirkung ausüben, die durch sogenannte Sonnenschutzfaktorzahlen, beispielsweise 1 - 15, erfaßbar ist. Diese Zahlen sollen die Benutzer von Sonnenschutzmitteln in die Lage versetzen, das Sonnenschutzmittel mit der richtigen Sonnenschutzfaktorzahl auszuwählen.

Um diese Auswahl zu erleichtern, werden neuerdings, beispielsweise in Freibädern an der Adria, täglich die UV-Strahlung und die entsprechende Sonnenschutzfaktorzahl als "Sonnenbrandwarnung" bekanntgegeben. Hierbei macht man sich ein Gerät - "Sun-Timer" - zunutze, welches - einem Belichtungsmesser ähnlich - die UV-Strahlen auffängt und elektronisch in Meßwerte umwandelt, denen die Sonnenschutzfaktorzahlen zugeordnet sind (Zeitschrift: HÖR ZU, Nr. 87/1984).

Zwar bringt diese Bekanntgabe der Sonnenschutzfaktorzahlen, die auch auf den Verpackungen von Sonnenschutzmitteln angegeben sind, vielen eine Erleichterung. Nachteilig ist aber, daß hierbei der jeweilige individuelle Zustand der Haut unberücksichtigt bleibt. Dieser wird in der Regel nach Gutdünken und nach oberflächlicher Inaugenscheinnahme beurteilt.

Voraussetzung für die richtige Auswahl eines Hautschutzmittels ist - wie sich gezeigt hat - nicht allein die objektiv gemessene UV-Strahlung, sondern auch eine Beurteilung des jeweiligen Zustandes der Haut.

Dieser ist grundsätzlich durch ihre subjektive, biologische Eigenart, beispielsweise hell und durchscheinend oder braun (pikmentiert) und undurchsichtig und zudem durch ihren Zustand infolge der Sonnen- bzw. UV-Strahlung gegeben. Beide Eigenarten bedingen ihren jeweiligen individuellen Bräunungswert. Nur dessen möglichst genaue Ermittlung kann eine sichere Grundlage für die richtige Auswahl des akut erforderlichen Sonnenschutzmittels geben.

Zwar sind mit den Sonnenschutzfaktorzahlen kombinierte Farbskalen zum Vergleich mit der jeweiligen Färbung der Haut bekannt geworden, so beispielsweise ein Verfahren und ein Gerät zur optischkolorimetrischen Ermittlung des Bräunungsgrades unterschiedlicher Hauttypen (DE-OS 20 58 579), mit welchem auf photomechanischem Wege die Ermittlung des Bräunungsgrades unterschiedlicher menschlicher Hauttypen möglich ist, was die Erstellung einer Bräunungsskala von 0,0 % bis 100 % zuläßt. Das Gerät ist so aufgebaut, daß es lediglich zur Messung des Bräunungsgrades der Haut eines Vergleichs mit einem von vielen Farbtönen bedarf, dem jeweils eine Zahl zugeordnet ist. Das Gerät besitzt eine Reihe von Farbtonträgern, die entweder als auf einer gemeinsamen Achse verschwenkbare Farb-Kunststoffplättchen oder Bestandteile einer Kunststoffscheibe ausgebildet sind. Es hat sich jedoch gezeigt, daß diese Farbvergleiche praktisch nicht zur hinreichend sicheren Auswahl des jeweils erforderlichen Sonnenschutzmittels führen können,

- 3 -

0193163

weil die Farbskalen die subjektiven Eigenarten einer Haut unberücksichtigt lassen.

Dem gegenüber liegt der Erfindung die Aufgabe zugrunde, eine elektrische Vorrichtung zu schaffen, mit der - in Abhängigkeit von den individuellen Eigenschaften der Haut und ihren jeweiligen Zuständen - die erforderliche Sonnenschutzfaktorzahl ermittelt werden kann, die die Auswahl des richtigen Sonnenschutzmittels ermöglicht.

Es wurde gefunden, daß sich diese Aufgabe in einfacher Weise dadurch lösen läßt, daß eine auf den Braunheitswert der Haut ansprechende opto-elektrische Sensoreinrichtung mit einer Lichtquelle und wenigstens ein Meßfotoelement einschließenden Reflexionslichtschranke zusammen mit einer Energiequelle und einem Ein-Ausschalter in einem Gehäuse angeordnet ist, in welchem die opto-elektrische Sensoreinrichtung über eine elektronische Auswertschaltung mit einer Anzeigeeinrichtung für die Sonnenschutzfaktorzahl in Schaltverbindung steht, und daß die Reflexionslichtschranke derart ausgebildet ist, daß nur rotes Licht auf die Haut geschickt wird.

Hiermit wird erfindungsgemäß nicht nur eine praktisch verwertbare Feststellung der individuell erforderlichen Sonnenschutzfaktorzahl ermöglicht, sondern in besonders vorteilhafter Weise erreicht, daß sich zugleich mit der Vorrichtung auch der Braunheitswert der Haut feststellen läßt, der beispielsweise zur individuellen Hautkontrolle, aber auch zu Vergleichen mit gebräunter Haut anderer herangezogen werden kann.

Dadurch, daß die Sensoreinrichtung im roten Spektralbereich arbeitet, ist die Vorrichtung nach der Erfindung in der Lage, von der Sonne gerötete oder gar infolge einer anderen Hautreizung rote Haut zu erfassen. Die Sensoreinrichtung liefert bei roter Haut Meßwerte, die auch bei weißer Haut zustandekommen. Beide Meßwerte - die von roter Haut und die von weißer Haut - werden von der elektronischen Auswertschaltung so verarbeitet, daß auf der Anzeigeeinrichtung diejenige Sonnenschutzfaktorzahl angezeigt wird, die für unbelichtete, also weiße, empfindliche Haut, notwendig ist.

Anders reagiert die Vorrichtung bei gebräunter bzw. brauner Haut, die keine Rot-Komponente aufweist. Hierfür wird der entsprechende Bräunungswert sowie die dazugehörige Sonnenschutzfaktorzahl angezeigt.

Bei einer Mischung brauner und roter Farbpigmente, d.h. bei einer sowohl gebräunten als auch rot gereizten Haut wird der Rotanteil erfaßt und Bestandteil des Meßwertes. Die Anzeigeeinrichtung zeigt die Sonnenschutzfaktorzahl an, die der Rötung der Haupt entspricht.

Die elektronische Auswertschaltung nach der Erfindung schließt eine Anordnung zur Festlegung eines unteren Schwellwertes entsprechend dem Braunheitswert einer extrem weißen Haut und eine entsprechende Anordnung zur Festlegung eines oberen Schwellwertes entsprechend dem Braunheitswert einer extrem braunen Haut ein. Hierdurch wird es möglich, trotz der Berücksichtigung unterschiedlicher Hauteigenschaften zu verwertbaren und objektiven Angaben für die Sonnenschutzfaktorzahlen zu kommen.

Die Ausführungsbeispiele der Erfindung verwenden eine Sensoreinrichtung, die nach den Ansprüchen 3 - 7 ausgebildet ist und eine künstliche Lichtquelle enthält, die für alle Messungen stets gleiches Licht liefert.

Die Vorrichtungen können in beliebig, jedoch entsprechend geformte Gehäuse, beispielsweise mit ovalem, rundem oder viereckigem Querschnitt untergebracht sein. Als Ein- und Ausschalter kann entweder ein handbetätigter Schalter am Gehäuse mit einem feststehenden und einem beweglichen Kontakt oder auch ein Hautkontaktschalter verwendet werden, dessen Kontakte einander gegenüberliegend entlang zweier Seitenkanten eines Meßfensters angeordnet sind.

Die elektronische Auswertschaltung schließt gemäß der Ansprüche 8 bis 13 einen Analog/Digitalwandler mit nachgeschaltetem, digitalem Umrechner für den Braunheitswert und für die Zugeordneten Schonnenschutzfaktorzahlen ein. In dieser Vorrichtung ist eine - vorzugsweise zweistellige - digitale Ziffernanzeige für den Braunheitswert und eine vorzugsweise digitale Ziffernanzeige für die Sonnenschutzfaktorzahlen vorgesehen.

In einem bevorzugten Ausführungsbeispiel ist die elektronische Auswertschaltung über eine handelsübliche Steuerschaltung an eine Leuchtdiodenkette angeschlossen.

Weitere Merkmale der Erfindung sind in den Ansprüchen 14 bis 16 gekennzeichnet.

Die Vorrichtung nach der Erfindung kann auch den Anschluß für eine Kette oder für ein Halsband aufweisen. Unter Einsatz üblicher, im Handel erhältlicher Schaltungselemente ist es möglich, die Vorrichtung etwas größer als eine normale Taschenuhr zu bauen. Bei Verwendung eines speziellen sogenannten User-Ships reduziert sich die Größe der Gesamtvorrichtung auf das Format einer üblichen Armbanduhr.

Die Ausführungsbeispiele werden nachfolgend anhand der Zeichnung erläutert.

Es zeigt:

Fig. 1    ein erstes Ausführungsbeispiel der Erfindung in perspektivischer Ansicht,

Fig. 2    einen Schnitt durch die Vorrichtung nach Fig. 1 parallel zur Grundfläche,

Fig. 3    einen Schnitt durch die Vorrichtung nach Fig. 1 senkrecht zur Grundfläche,

Fig. 4    ein vereinfachtes Schaltbild zur Darstellung der einzelnen Meßmöglichkeiten des Braunheitswertes,

Fig. 5    ein vereinfachtes Blockschaltbild des ersten Ausführungsbeispieles,

Fig. 6    ein vereinfachtes Blockschaltbild einer abgewandelten Ausführungsform des ersten Ausführungsbeispieles,

Fig. 7    schematisch die Draufsicht auf ein zweites Ausführungsbeispiel,

Fig. 8    ein vereinfachtes Blockschaltbild für das zweite Ausführungsbeispiel,

Fig. 9    ein vereinfachtes Blockschaltbild einer abgewandelten Schaltung für das zweite Ausführungsbeispiel,

Fig. 10   eine perspektivische Teilschnittansicht eines bevorzugten Ausführungsbeispieles,

Fig. 11   ein vereinfachtes Blockschaltbild für das Ausführungsbeispiel nach Fig. 10 und

Fig. 12   einen Querschnitt durch ein in Verbindung mit allen Ausführungsbeispielen einsetzbares lichtsammelndes Gehäuse.

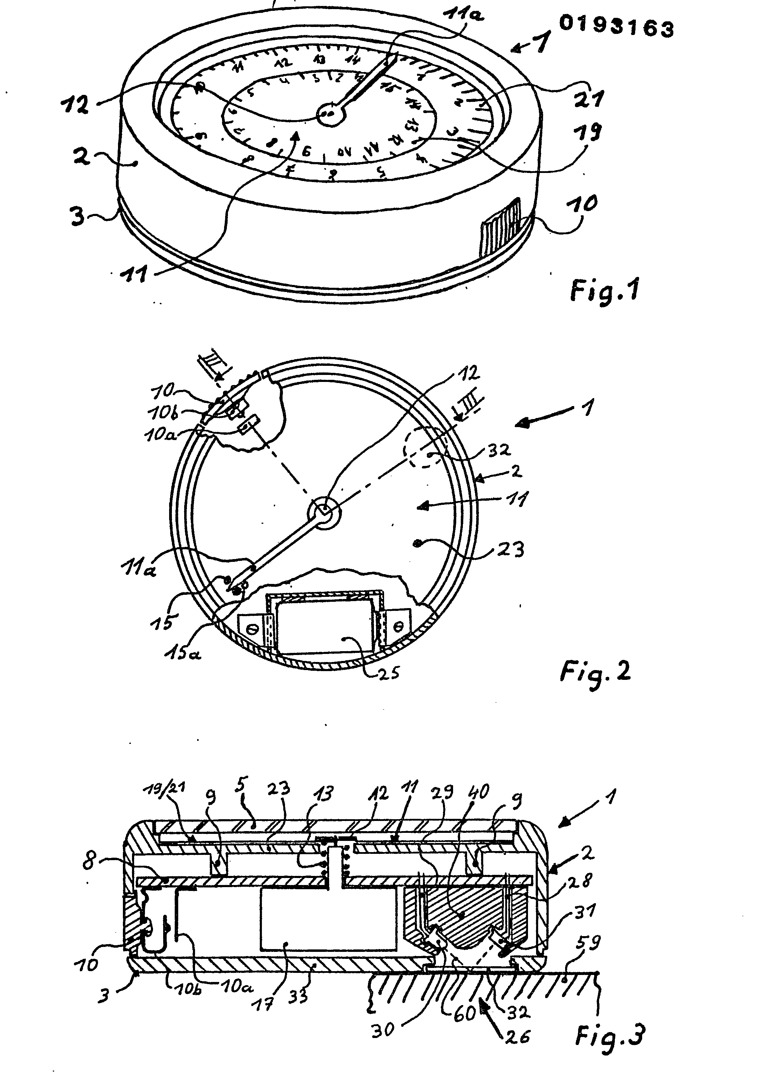

Zur Erläuterung des ersten Ausführungsbeispieles der Erfindung wird auf die Fig. 1 - 6 Bezug genommen.

Fig. 1 zeigt eine perspektivische Ansicht eines Gehäuses 1 für das erste Ausführungsbeispiel der Erfindung. Der Durchmesser des Gehäuses des dargestellten Ausführungsbeispieles liegt etwa zwischen 5 und 8 cm, die Höhe beträgt ca. 1 - 3 cm.

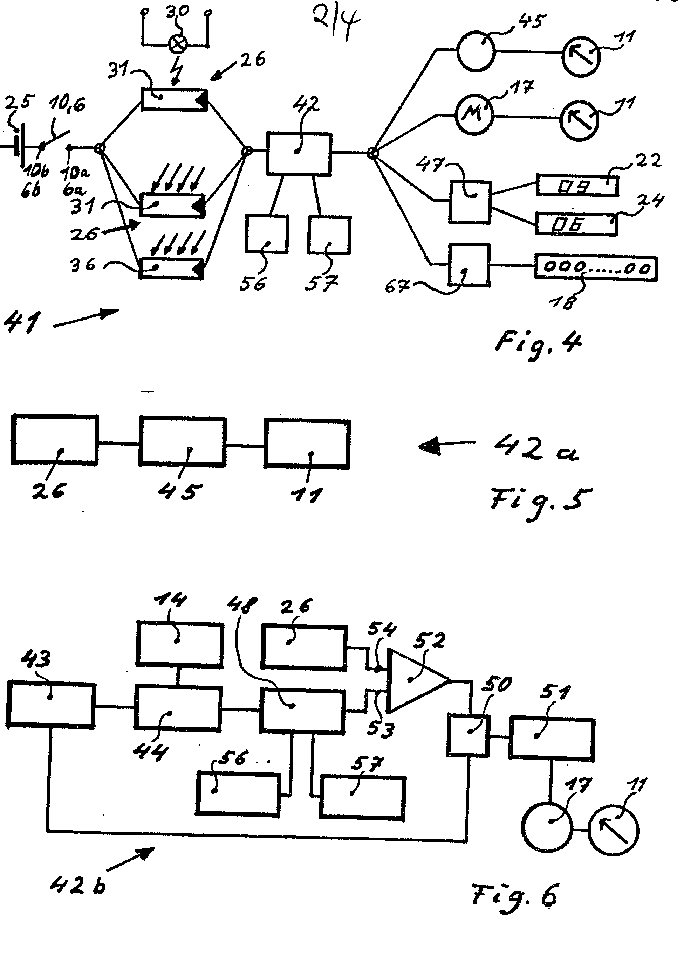

Fig. 2 zeigt einen Schnitt durch das Gehäuse 1 in einer Ebene parallel zur Bodenfläche, wo hingegen Fig. 3 einen Schnitt senkrecht zu dieser Ebene und zwar entlang der Linie III, III in Fig. 2 darstellt.

Das zylindrische Gehäuse besitzt eine Seitenwand 2, eine untere Gehäuseabdeckung 33 sowie eine obere transparente Abdeckung 5. Unterhalb dieser transparenten Abdeckung 5 ist der Zeiger 11a einer Zeigerrundanzeige 11 erkennbar, der mit zwei Skalen in Anzeigeverbindung steht. Eine erste, innen liegende Skala 19 gibt die Sonnenschutzfaktorzahlen 1 - 15 an, die in linearer Aufteilung am Umfang der Scheibe angeordnet sind.

Die zweite Skala trägt Zahlenangaben in beliebiger Unterteilung zur Angabe des Braunheitswertes der Haut. Die Angaben der ersten Skala 19 sind den Zahlenangaben auf der zweiten Skala 21 zugeordnet. Die Seitenwand 2 des Gehäuses 1 wird durch eine untere Kante 3 abgeschlossen. In der Seitenwand 2 ist die Handbetätigung eines allgemein mit 10 bezeichneten Ein-Ausschalters erkennbar.

Auf eine in Fig. 1 nicht dargestellte Weise kann das Gehäuse 1 mit einem Haken zum Anbringen einer Halskette bzw. einer Schnur ausgerüstet sein.

Einzelheiten des Gehäuses sind den Fig. 2 und 3 entnehmbar. Die kreisförmige transparente Abdeckung 5 ist im oberen Bereich des Gehäuses 1 gelagert. Die untere Gehäuseabdeckung 33 kann in an sich bekannter Weise mit der Seitenwand 2 verschraubt, verklebt ider verklemmt sein, um Zugang zu dem Inneren des Gehäuses zu ermöglichen.

Der Ein-Ausschalter 10 läßt sind in Draufsicht der Fig. 2 und in Seitenansicht der Fig. 3 entnehmen. Das Handbetätigungsglied ist mit einem beweglichen Kontakt 10b verbunden, der einem feststehenden Kontakt 10a gegenüberliegt. Ohne am Kern der Erfindung etwas zu ändern, kann auch jeder beliebige andere Ein-Ausschalter zum Einsatz kommen.

Unterhalb der transparenten Abdeckung 5 ist ein Skalenträger 23 erkennbar, der vermittels Abstandsbolzen 9 mit einer Platine 8 verbunden ist. Auf dem Skalenträger 23 sind die beiden Skalen 19 und 21 vorgesehen, die in bevorzugten Ausführungsbeispielen auf selbstklebendem Papier aufgebracht sind und mit dem Skalenträger 23 verklebt werden. Diese lösbare Befestigung der Skalen auf dem Skalenträger 23 hat den Vorteil, daß die Vorrichtung rasch auf andere Skalenteilungen umgerüstet oder bei der Herstellung mit unterschiedlichen Skalen versehen werden kann. Die Skalen können aber auch direkt auf dem Skalenträger 23 aufgebracht, beispielsweise aufgedruckt werden.

Innerhalb der unteren Gehäuseabdeckung 33, vorzugsweise im Randbereich, ist eine Reflexlichtschranke 26 für einen in den Fig. 1 und 2 nicht dargestellten Meßwertgeber erkennbar. Die Reflexlichtschranke 26 besteht im dargestellten Ausführungsbeispiel aus einer Leuchtdiode 30 und einem Meßfotoelement bzw. Meßfotowiderstand 31, die beide so ausgerichtet sind, daß ein von der Leuchtdiode 30 ausgesandter Lichtstrahl 60 durch ein Meßfenster 32 auf einen Hautbereich 59 gerichtet und von diesem reflektiert werden kann. Die Leuchtdiode 30 und der Meßfotowiderstand 32 können auf einer gemeinsamen Halterung 40 sitzen, die dem Meßfenster 32 gegenüber angeordnet ist. Der Leuchtdiode 30 wird Strom über Anschlußdrähte 29 zugeführt, wo hingegen die Signale des Meßfotoelementes bzw. Meßfotowiderstandes 31 über Anschlußdrähte 28 einer später erläuterten elektronischen Schaltung zugeführt werden.

Der Zeiger 11a ist mit einem Zeigerauge 12 versehen, welches auf einem Drehstift angeordnet ist, um den herum eine zentrale Kontaktfeder 13 ausgespannt ist, deren Bedeutung später erläutert wird.

Im Schnittbild der Fig. 2 sind Skalenträger 23 und das darunterliegende Meßfenster 32 erkennbar. Der Zeiger 11a kann entweder, wenn er aus leidendem Material besteht, einen Null-Kontakt 15 betätigen oder, wenn er aus Kunststoff bzw. nichtleitendem Material besteht, eine Reflexlichtschranke 15 betätigen.

Im unteren Bereich der Fig. 2 ist eine Energiequelle, beispielsweise eine 6 bzw. 9 Volt Batterie 25 erkennbar, die auf übliche Weise in einer an sich bekannten Klemmhalterung sitzt.

Fig. 4 zeigt in einer Übersicht die Möglichkeiten, mit denen ein Meßwert erzeugt, elektronisch verarbeitet und dargestellt werden kann.

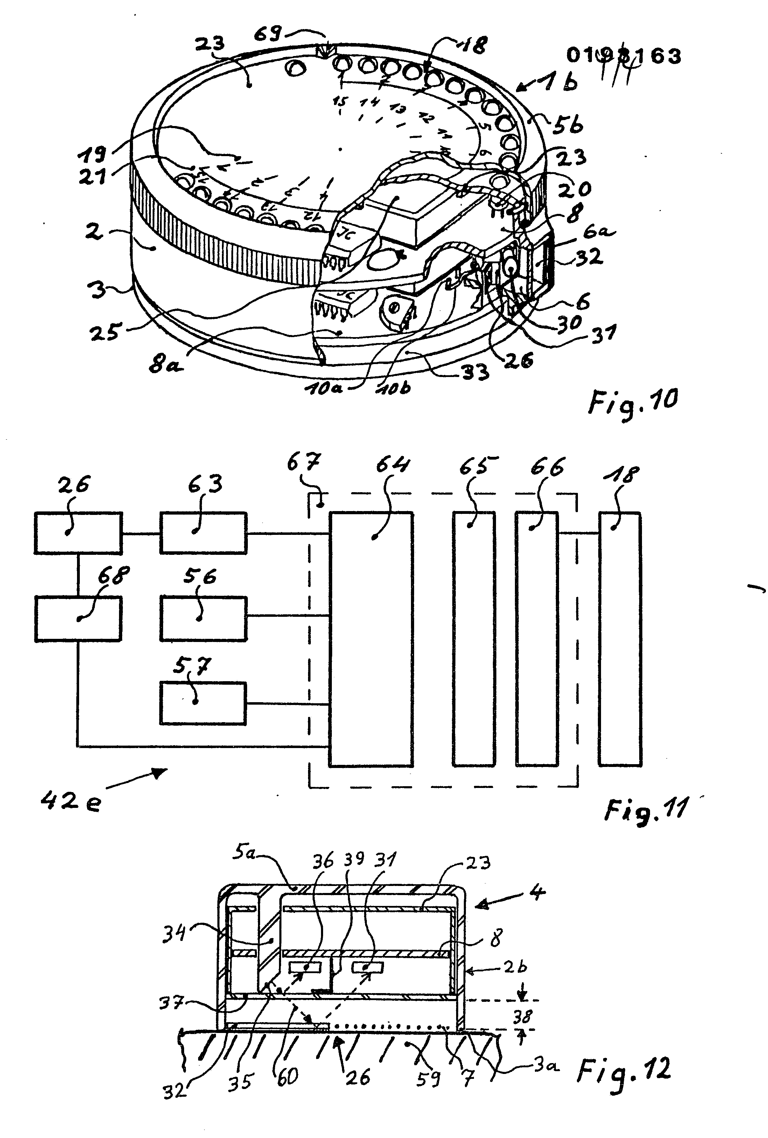

In Reihe mit der Energiequelle 25 ist der Ein-Ausschalter 10 erkennbar, der zusammen mit dem Meßfenster 32 gemäß Fig. 10 eine integrierte Einheit 6 mit Hautkontakten 6a,6b entlang der Seitenkanten des Meßfensters 32 bilden kann.

Im oberen Bereich der Fig. 4 ist ein Meßwertgeber 26 erkennbar, der als Meßfotoelement bzw. als Meßfotowiderstand 31 ausgebildet sein kann. Ihm gegenüber sitzt die bereits erläuterte Leuchtdiode 30, die beispielsweise eine rotleuchtende Leuchtdiode, vorzugsweise mit einer Wellenlänge von 600 nm bis 750 nm oder auch eine Miniaturglühlampe sein kann.

Im unteren Bereich der Fig. 4 ist das Meßfotoelement bzw. der Meßfotowiderstand 31 in Verbindung mit einem Referenzfotoelement bzw. Referenzfotowiderstand 36 dargestellt. Diese Kombination wird benötigt, wenn als Lichtquelle das Umgebungslicht verwendet wird, welches durch ein lichtsammelndes Gehäuse 4 gemäß der später erläuterten Fig. 12 einem Lichtleiter 34 zugeführt wird.

Gemäß dem elektronischen Prinzipschaltbild 41 der Fig. 4 schließt sich an den Meßwertgeber 26 eine elektronische Auswertschaltung 42 an, an welche die unterschiedlichen Anzeigemöglichkeiten angeschlossen sind.

Im oberen Bereich ist ein Drehspulmeßwerk 45 erkennbar, welches mit einer Zeigerrundanzeige 11 in Wirkverbindung steht.

Darunter ist ein Schrittmotor 17 angedeutet, der ebenfalls auf eine Zeigerrundanzeige 11 einwirkt.

Eine andere Darstellungsmöglichkeit der Meßwerte besteht darin, daß die elektronische Auswertschaltung 42 mit einem digitalen Umrechner 47 verbunden wird, an welchen zweistellige Digitalanzeigen 22 bzw. 24 angeschlossen sind.

Bei einem bevorzugten Ausführungsbeispiel wird zur Anzeige eine Leuchtdiodenkette 18 eingesetzt, die über eine Ansteuerschaltung 67 mit der elektronischen Auswertschaltung 42 verbunden ist.

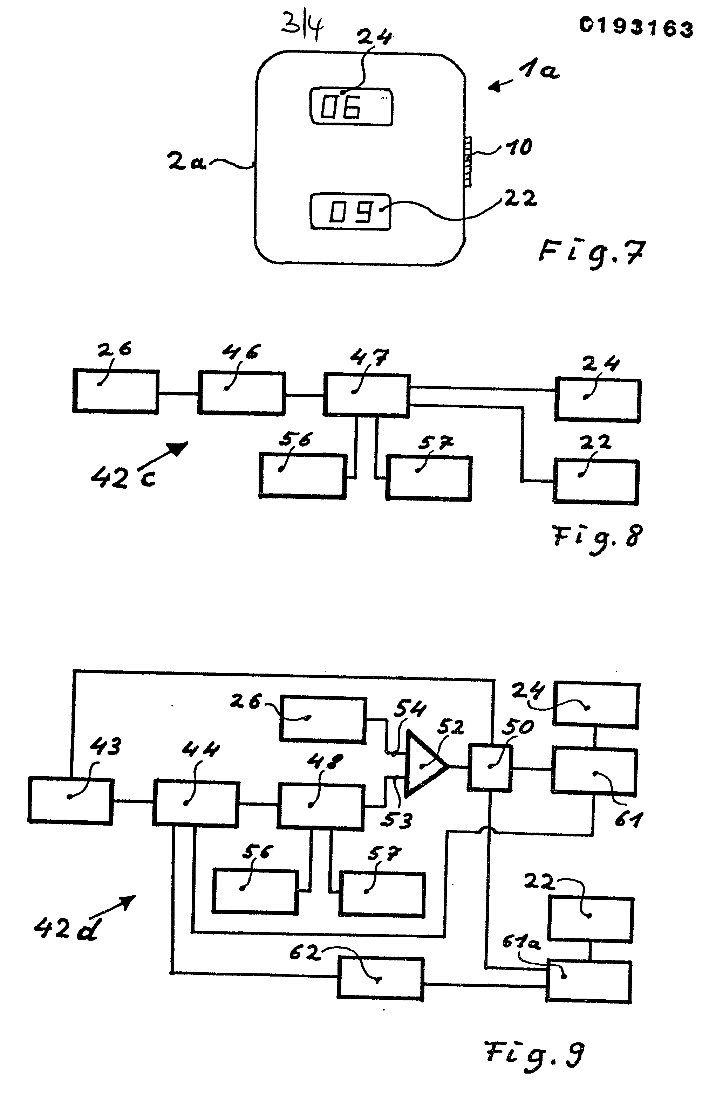

Fig. 5 zeigt ein vereinfachtes Blockschaltbild einer elektronischen Auswertschaltung 42a für ein Drehspulmeßwerk 45, welches mit einer Zeigerrundanzeige 11 verbunden ist. Hiernach wird einfach der Meßwertgeber 26 an das Drehspulmeßwerk 45 angeschlossen, welches auf die Zeigerrundanzeige 11 einwirkt.

Eine abgewandelte elektronische Auswertschaltung 42b für ein Zeigerrundinstrument 11 ist in Form eines vereinfachten Blockschaltbildes in Fig. 6 dargestellt. Der Meßwertgeber 26 ist an einen zweiten Eingang 54 eines Komparators 52 angeschlossen. An einem zweiten Eingang 53 des Komparators 52 liegt ein Vergleichspannungsgeber 48 an. Dieser Vergleichsspannungsgeber ist mit einem Zähler bzw. Schieberegister 44 verbunden, welcher von einem üblichen Oszillator 43 angetrieben wird. Der Zähler bzw. das Schieberegister 44 ist mit einer Nullpunkt-Resetschaltung 14 verbunden, die - beispielsweise über den Null-Kontakt 15 bzw. die Reflexlichtschranke 15a - eine Rückstellung des Zählers bzw. Schieberegisters 44 auf Null nach beendeter Messung bewirkt.

Jedes Ausführungsbeispiel der Erfindung weist eine Anordnung 56 zur Festlegung eines oberen Schwellwertes und eine Anordnung 57 zur Festlegung eines unteren Schwellwertes auf, wie dieses in Fig. 4 angedeutet ist. Mit einer Anordnung 56 kann ein unterer Schwellwert entsprechend dem Braunheitswert einer extrem weißen Haut festgelegt werden, wo hingegen mit einer Anordnung 57 ein oberer Schwellwert für den Braunheitswert einer extrem braunen Haut festgelegt werden kann.

Im Ausführungsbeispiel einer elektronischen Auswertschaltung gemäß Fig. 6 sind die Anordnungen 56 und 57 als Widerstände für den Vergleichsspannungsgeber 48 ausgebildet. Im einzelnen treibt das Schieberegister bzw. der Zähler 44 den Vergleichsspannungsgeber 58 an, der aus an den Ausgängen des Zählers bzw. des Schieberegisters 44 anliegenden Widerständen besteht. Diese Widerstände des Vergleichsspannungsgebers 48 sind so bemessen und geschaltet, daß am ersten Eingang 53 des Komparators 52 eine Treppenkurve mit vorzugsweise 64 Spannungsstufen anliegt. Sobald eine dieser Spannungsstufen der vom Meßwertgeber 26 an den Eingang 54 des Komparators 52 abgegebenen Spannung entspricht, schaltet der Komparator auf an sich bekannte Weise über einen Meßwerthaltestromkreis 50 eine Schrittschaltsteuerung 51 für den Schrittmotor 17 ab, der direkt vom Oszillator 43 geschaltet wird. Der Schrittmotor betätigt eine Zeigerrundanzeige 11.

Fig. 7 zeigt in Draufsicht ein viereckiges Gehäuse 1a mit einer entsprechend ausgebildeten Seitenwand 2a und Digitalanzeigen 22 und 24. Die Digitalanzeige 22 ist zweistellig und zeigt die Sonnenschutzfaktorzahlen 1 - 15 an. Mit der Digitalanzeige 24 wird der Braunheitswert der Haut angezeigt.

Fig. 8 zeigt in Übereinstimmung mit Fig. 4 ein vereinfachtes Blockschaltbild einer elektronischen Auswertschaltung 42c. Der Meßwertgeber 26 liegt über einen A/D-Wandler 46, vorzugsweise mit Sample and Hold-Schaltkreis, am digitalen Umrechner 47 an, der einerseits mit Anordnungen 56 und 57 zur Festlegung eines oberen bzw. unteren Schwellwertes verbunden ist und mit den Digitalanzeigen 24 und 22 in Schaltverbindung steht.

Im vereinfachten Blockschaltbild nach Fig. 9 einer elektronischen Auswertschaltung 42d für die Digitalanzeigen 22 und 24 ist ein Vergleichsspannungsgeber 48 zusammen mit dem Meßwertgeber 26 über die Eingänge 53 und 54 des Koparators 52 an den Meßwerthaltestromkreis 50 angeschlossen. Dieser steht über einen Anzeigetreiber 61 mit Dezimal-Kodierer mit der Digitalanzeige 24 und über einen Anzeigentreiber 61a mit Dezimal-Kodierer mit der zweistelligen Digitalanzeige 22 in Schaltverbindung. Die Anzeigentreiber, 61 direkt und 61a über einen Umrechner 62, sind mit dem Zähler bzw. Schieberegister 44 verbunden, welches, wie im Ausführungsbeispiel nach Fig. 6, von dem Oszillator 43 angetrieben wird.

Für die einzelnen Schaltungselemente 43, 44, 46, 47, 48 sowie 52, 53 und 54 und die Anordnungen 56 und 57, aber auch für den Meßwerthaltestromkreis 50, werden handelsübliche Schaltungselemente eingesetzt.

Fig. 10 zeigt ein bevorzugtes Ausführungsbeispiel der Erfindung, bei welchem in einem Gehäuse 1b eine Leuchtdiodenkette 18 vorgesehen ist, die mit einer ersten Skala 19 zur Anzeige der Sonnenschutzfaktorzahlen und einer zweiten Skala 21 zur Anzeige der Braunheitswertzahlen in Anzeigeverbindung steht. Die Leuchtdioden 20 der Leuchtdiodenkette 18 sind auf einer Platine 8 angeordnet.

Im Ausschnitt des Gehäuses 1b sind die Energiequelle 25 sowie der Skalenträger 23 für die Skalen 19 und 21 erkennbar.

Fig. 10 zeigt weiterhin die Platine 8 und eine weitere Platine 8a, welche die einzelnen elektronischen Schaltelemente aufnehmen. Die untere Gehäuseabdeckung 33 weist eine untere Kante 3 auf. Deutlich ist die aus dem Meßfenster 32 und dem Ein-Ausschalter 6 integrierte Einheit erkennbar, die auch die Leuchtdiode 30 des Meßwertgebers 26 und ein Meßfotoelement bzw. Meßfotowiderstand 31 einschließt. Die transparente Abdeckung 5b kann drehbar und mit einer Marke 69 zum Festhalten eines Meßwertes kombiniert sein. Normalerweise wird eine feste Abdeckung 5 verwendet.

Die elektronische Auswertschaltung 42e für die Leuchtdiodenkette 18 ist in Fig. 11 dargestellt. Sie besteht aus einer Ansteuerschaltung 67 mit einem Schaltkreis 64, in welchem ein Vergleichsspannungsgeber 48 und der Komparator 52 zusammengefaßt sind. An den Schaltkreis 64 schließt sind eine Logikschaltung 65 zum Ansteuern der Leuchtdiodenkette 18 über eine Treiberschaltung 66 an- Die Schaltungsanordnung 64 steht über einen automatischen Halte/Abschaltstromkreis 68 mit definierter Haltezeit mit dem Meßwertgeber 26 in Schaltverbindung. Zusätzlich ist an den Stromkreis 64 eine Schaltungsanordnung 63 zum Halten eines analogen Meßwertes angeschlossen.

Fig. 12 schließlich zeigt einen Querschnitt durch ein lichtsammelndes Gehäuse 4 mit einer transparenten Abdeckung 5a, welche in einen Lichtleiter 34 mit einer Lichtaustrittsfläche 35 mündet. Innerhalb des lichtleitenden Gehäuses ist ein Skalenträger 23 und eine Platine 8 angedeutet. Die Seitenwand 2b endet in einer unteren Kante 3a, die auf dem Hautbereich 59 aufsteht.

In einem definierten Abstand 38 vom Hautbreich 59 bzw. der unteren Kante 3a ist ein halbdurchlässiger Spiegel 37 vorgesehen, der einerseits den Lichtstrahl 60 zum Meßfotowiderstand 31 durchläßt, zum anderen aber einen Anteil des Lichtstrahls 60 zum Referenzfotoelement bzw. Referenzfotowiderstand 36 reflektiert. Beide Meß- bzw. Referenzlichtaufnehmer sind durch eine Trennwand 39 getrennt. Auch das lichtsammelnde Gehäuse 4 ist mit einem Meßfenster 32 versehen, welches als Spektralfilter, vorzugsweise als entspiegeltes Linienfilter im roten Spektralbereich ausgebildet sein kann. Das lichtsammelnde Gehäuse 4 bzw. der Lichtleiter 34 können rot eingefärbt sein.

Der halbdurchlässige Spiegel 37 kann ein Teil der unteren Abdeckung 33 des Gehäuses 4 sein. Als untere Gehäuseabdeckung kann auch eine Scheibe aus Gittermaterial 7 eingesetzt sein. Als Steuerschaltung 67 kann eine handelsübliche Steuerschaltung, z.B. ein IC U 170, U 180 bzw. U 1096 verwendet werden. Das Meßfenster 32 kann insgesamt durch die Scheibe 7 aus Gittermaterial abgedeckt sein bzw. aus entspiegeltem Glas bzw. entspiegeltem transparenten Kunststoff bestehen.

- 14 -

Liste der verwendeten Bezeichnungen

1 Gehäuse

1a Gehäuse

1b Gehäuse

2 Seitenwand für ein zylindrisches Gehäuse

2a Seitenwand für ein Gehäuse mit viereckigem Querschnitt

3 untere Kante

3a untere Kante

4 lichtsammelndes Gehäuse

5 transparente Abdeckung

5a transparente Abdeckung

5b transparente Abdeckung (drehbar)

6 Ein-Ausschalter mit integriertem Meßfenster

7 Scheibe aus Gittermaterial

8 Platine

8a Platine

9 Abstandsbolzen

10 Ein-Ausschalter

10a feststehender Kontakt

10b beweglicher Kontakt

11 Zeigerrundanzeige

11a Zeiger

12 Zeigerauge

13 zentrale Kontaktfeder

14 Nullpunkt-Resetschaltung

15 Null-Kontakt

15a Reflexlichtschranke vom Zeiger 11a schaltbar

16    .

17 Schrittmotor

18 Leuchtdiodenkette

19 Skala für Sonnenschutzfaktorzahlen, erste Skala

20 Leuchtdiode der Leuchtdiodenkette

21 Skala für Braunheitsgradzahlen, zweite Skala

22 Digitalanzeige, zweistellig

23 Skalenträger

24 Digitalanzeige, dreistellig mit einer Dezimalen

25 Energiequelle, Batterie 6 Volt bzw. 9 Volt

26 Meßwertgeber

27

28 Anschlußdrähte

29 Anschlußdrähte

30 Leuchtdiode des Meßwertgebers

31 Meßfotoelement, Meßfotowiderstand

32 Meßfenster

33 untere Gehäuseabdeckung

34 Lichtleiter

35 Lichtaustrittsfläche

36 Referenzfotoelement, Referenzfotowiderstand

37 halbdurchlässiger Spiegel

38 definierter Abstand

39 Trennwand

40 gemeinsame Halterung, Aufnahme

41 elektronische Prinzip-Schaltungsanordnung

42 elektronische Auswertschaltung

42a Auswertschaltung für ein Drehspulinstrument

42b Auswertschaltung für ein Zeigerrundinstrument

42c Auswertschaltung für Digitalanzeigen mit A/D Wandler

42d Auswertschaltung für eine Digitalanzeige

42e Auswertschaltung für eine Leuchtdiodenkette

43 Oszillator

44 Zähler bzw. Schieberegister

45 Drehpulsmeßwerk

46 A/D Wandler, vorzugsweise mit sample and hold Schaltkreis

47 digitaler Umrechner

48 Vergleichsspannungsgeber

49

50  Meßwerthaltestromkreis

51 Schrittschaltsteuerung

52 Komparator

53 erster Eingang

54 zweiter Eingang

55

56 Anordnung zur Festlegung eines oberen Schwellwertes

57 Anordnung zur Festlegung eines unteren Schwellwertes

58

59 Hautbereich der Messung

60 Lichtstrahl

61 Anzeigentreiber mit Dezimalkodierer

61a Anzeigentreiber mit Dezimalkodierer

62 Umrechner

63 Schaltungsanordnung zum Halten eines analogen Meßwertes

64 Schaltkreis mit zusammengefaßtem Vergleichsspannungsgeber 48
   und Komparator 52

65 Logikschaltung zum Ansteuern der Leuchtdiodenkette

66 Treiberschaltung für die Leuchtdiodenkette

67 Ansteuerschaltung für die Leuchtdiodenkette

68 automatischer Halte/Abschaltstromkreis mit definierter
   Haltezeit

69 Marke

70

- 14 -

Patentansprüche

1. Vorrichtung zur Ermittlung einer jeweils akuten Sonnenschutzfaktorzahl zwecks Auswahl des zum Schutz der Haut geeigneten
Sonnenschutzmittels,
dadurch gekennzeichnet,
daß eine auf den Braunheitswert der Haut (59) ansprechende opto-
elektrische Sensoreinrichtung (26, 30, 31, 32, 40) mit einer
eine Lichtquelle (30) und wenigstens ein Meßfotoelement (31)
einschließenden Reflexionslichtschranke (26) zusammen mit einer
Energiequelle (25) und einem Ein-Ausschalter (10, 6) in einem
Gehäuse (1, 1a, 1b) angeordnet ist, in welchem die opto-elektrische Sensoreinrichtung über eine elektronische Auswertschaltung (41, 42, 42a bis e) mit einer Anzeigeeinrichtung
(22, 28, 29, 21, 22, 24) für die Sonnenschutzfaktorzahl in
Schaltverbindung steht, und daß die Reflexionslichtschranke
(26) derart ausgebildet ist, daß nur rotes Licht auf die Haut
(59) geschickt wird.

2. Vorrichtung nach Anspruch 1, dadurch gekennzeichnet, daß die
elektronische Auswertschaltung (41, 42, 42a bis e) eine Anordnung (56) zur Festlegung eines unteren Schwellwertes entsprechend dem Braunheitswert einer extrem weißen Haut und
eine Anordnung (57) zur Festlegung eines oberen Schwellwertes
für den Braunheitswert einer extrem braunen Haut einschließt.

3. Vorrichtung nach einem der Ansprüche 1 oder 2, dadurch gekennzeichnet, daß die Sensoreinrichtung (26, 30, 31, 32) einem Meßfenster (32) in einem dem zu untersuchenden Hautbereich (59) zugewandten Gehäuseteil (2, 33) gegenübersitzt und daß das Meßfenster (32) und der Ein-Ausschalter (6) zu einem Bauteil integriert sind.

4. Vorrichtung nach einem der Ansprüche 1 - 3, dadurch gekennzeichnet, daß die Lichtquelle eine rotleuchtende Leuchtdiode (30) ist.

5. Vorrichtung nach Anspruch 4, dadurch gekennzeichnet, daß die Wellenlänge des von der Leuchtdiode ausgesandten Lichtes zwischen 600 nm und 750 nm liegt.

6. Vorrichtung nach einem der Ansprüche 1 - 3, dadurch gekennzeichnet, daß die Lichtquelle (30) eine Miniaturglühlampe ist und daß das Meßfenster (32) mittels eines im roten Spektralbereich durchlässigen Spektralfilters abgeschlossen ist.

7. Vorrichtung nach einem der Ansprüche 1 - 6, dadurch gekennzeichnet, daß im Gehäuse (1, 1a, 1b) eine gemeinsame Halterung (40) für die Lichtquelle (30) und die Meßfotoelemente (31) der Reflexionslichtschranke (26) vorgesehen ist.

8. Vorrichtung nach einem der Ansprüche 1 - 7, dadurch gekennzeichnet, daß die elektronische Auswertschaltung (42a) an ein Drehspulmeßwerk (45) mit einer Zeigerrundanzeige (11) angeschlossen ist, deren - vorzugsweise in der Meßstellung - arretierbarer - Zeiger (11a) mit einer ersten Skala (19) für die Sonnenschutzfaktorzahlen und einer zweiten Skala (21) für den Braunheitswert der Haut in Anzeigeverbindung steht.

9. Vorrichtung nach einem der Ansprüche 1 - 5, dadurch gekennzeichnet, daß die elektronische Auswertschaltung (42c) einen Analog-Digitalwandler (46) mit nachgeschaltetem, digitalen Umrechner (47) für den Braunheitswert und für die zugeordnete Sonnenschutzfaktorzahl einschließt und an eine digitale Ziffernanzeige (24) für den Braunheitswert und eine - vorzugsweise zweistellige - digitale Ziffernanzeige (22) für die Sonnenschutzfaktorzahlen angeschlossen ist.

10. Vorrichtung nach einem der Ansprüche 1 - 7, dadurch gekennzeichnet, daß die elektronische Auswertschaltung (42e) über eine handelsübliche Steuerschaltung (67) an eine Leuchtdiodenkette (18) angeschlossen ist.

11. Vorrichtung nach Anspruch 10, dadurch gekennzeichnet, daß zwischen der Steuerschaltung (67) und dem Meßfotoelement (31) eine analoge Meßwerthalteschaltung (63) vorgesehen ist.

12. Vorrichtung nach einem der Ansprüche 10 oder 11, dadurch gekennzeichnet, daß zwischen dem Meßfotoelement (31) und der Steuerschaltung (67) ein automatischer Abschaltstromkreis (68) vorgesehen ist.

13. Vorrichtung nach einem der Ansprüche 10 - 12, dadurch gekennzeichnet, daß die Leuchtdiodenkette (18) der Umfangsform des Gehäuses (1, 1a) angepaßt ist und ihre Leuchtdioden (20) einer ersten Skala (19) für die Sonnenschutzfaktorzahlen und einer zweiten Skala (21) für die Braunheitswerte des Hautbereiches (59) zugeordnet sind.

14. Vorrichtung nach einem der Ansprüche 1, 8 oder 13, dadurch gekennzeichnet, daß die Skalen (19, 21) aus selbstklebendem Material bestehen und auswechselbar auf einem Skalenträger (23) angeordnet sind.

15. Vorrichtung nach Anspruch 1, dadurch gekennzeichnet, daß die Öffnung im Gehäuse (1, 1a, 1b) durch die das Licht (60) der Lichtquelle auf die Haut (59) geschickt wird, mittels entspiegeltem Glas oder entspiegeltem transparentem Kunststoff
oder einer Scheibe (7) aus Gittermaterial abgeschlossen ist.

16. Vorrichtung nach einem oder mehreren der Ansprüche 1 - 17,
    dadurch gekennzeichnet, daß der Ein-Ausschalter (6) Kontakte
    (6a, 6b) aufweist, die entlang zweier gegenüberliegender Ränder des Meßfensters (32) angeordnet und als Kontakte eines
    Hautkontaktschalters ausgebildet sind.

Fig.1

Fig.2

Fig.3

0193163

Fig. 4

Fig. 5

Fig. 6

3/4

0193163

24

06

1a

2a

10

09

22

Fig.7

26 46 47 24

56 57 22

42c

Fig.8

26 54 52 50 24

43 44 48 53 61

56 57

42d

22

61a

62

Fig.9

Fig.10

0193163

Fig.11

Fig.12